# EUROPEAN PATENT APPLICATION

(11) **EP 0 974 361 A1**
(43) Date of publication of application: **26.01.2000**
(21) Application number: 97902609.3
(22) Date of filing: 07.02.1997
(51) Int. Cl.: A61K 45/00

(54) **METHOD OF USING ANTACID AGENT AND PHARMACEUTICAL PREPARATION CONTAINING ANTACID AGENT**

(30) Priority: 08.02.1996 JP 5818196
(71) Applicant: CHUGAI SEIYAKU KABUSHIKI KAISHA, Tokyo 115 (JP)
(72) Inventor: SHINKAI, Chie Chugai Seiyaku Kabushiki Kaisha, Tokyo 171 (JP); OZAWA, Koichi Chugai Seiyaku Kabushiki Kaisha, Tokyo 171 (JP)
(74) Representative: VOSSIUS & PARTNER
(86) International application number: JP9700307
(87) International publication number: WO9728823

(57) **Abstract**

A method of using an antacid for preventing sucralfate from adsorbing or trapping another drug, as well as a pharmaceutical preparation comprising sucralfate, another drug and an antacid such that the another drug is prevented from being adsorbed on or trapped by sucralfate are disclosed.

The use of the antacid in an environment where sucralfate is used along with another drug can prevent the occurrence of interactions between sucralfate and the another drug such as inhibited or delayed absorption of the drug and the decrease in its efficacy.

## Description

### TECHNICAL FIELD

This invention relates to a method of using an antacid for preventing sucralfate from adsorption or trapping of another drug, thereby ensuring that the inherent absorption characteristics of the drug can be maintained. The invention also relates to a pharmaceutical preparation for the stated purpose that comprises sucralfate, another drug and an antacid.

### BACKGROUND ART

Sucralfate is an anti-ulcer agent having a mucosa protecting action and currently finds extensive use in clinical applications. When administered into the digestive tract, sucralfate reacts with gastric acid to form a gel which, by means of binding with plasma protein, physical adhesion and so forth, adheres to the mucous membrane of the digestive tract, particularly its ulcer site, to create a protective barrier. At the ulcer site, the barrier protects the ulcerated surface from the attack of gastric acid, pepsin and so forth and promotes the ability of the human body to repair the mucous membrane of the stomach, thereby exhibiting a healing effect.

On account of its protective action, limited side effects and other advantages, sucralfate is often used along with various drugs in clinical applications. However, there have been reported cases of sucralfate interaction with the drugs used concurrently such as impaired or retarded absorption of the drugs and the drop in their efficacy and the reason is believed to be the adsorption of the drug on the sucralfate in the digestive tract, its trapping in the gel or the formation of a complex which also contributes to suppressed absorption. To mention a few examples of these cases, Giesing et al. reported that digoxin experienced a 19% drop in adsorption, and requiring an interval of at least 2 hours between the administrations of the two drugs (Gastroenterology, 84:1165. 1983); Goss et al. reported that the bioavailability of ketoconazole dropped by 21% when it was used along with sucralfate and that therefore it should not be administered simultaneously with sucralfate (Clin. Pharmacol. Ther., 49:128, 1991); Anaya et al. reported that the absorption of ibuprofen was reduced and retarded by its use along with sucralfate (Biopharm. Drug Dispos., 7:443, 1986); Maconochie et al. reported that the bioavailability of ranitidine dropped by 29% when it was used along with sucralfate (Clin. Pharmacol. Ther., 41:205, 1987); and Yoshida et al. reported the drop in cimetidine's AUC caused by simultaneous administration of sucralfate (Journal of the Society of Gastrointestinal Diseases of Japan, 84:1025, 1987). Many other reports have been published on side effects such as retarded drug absorption that were caused by simultaneous use of sucralfate.

The common methods that are currently employed at the clinical setting to deal with the suppressed absorption of drugs due to the simultaneous use of sucralfate are limited to either avoiding concurrent use of sucralfate on sequentially administering the two drugs with a certain time interval being allowed. Thus, few practical approaches have been proposed that enable simultaneous administration of drugs with sucralfate and which yet secure the high bioavailability of the drugs.

The present inventors conducted intensive studies in order to develop a method for preventing sucralfate from suppressing the absorption of other drugs and they unexpectedly found that when an antacid was used, drugs could be rapidly released without being adsorbed on or trapped by a gel of sucralfate, whereby the inherent absorbability of the drugs was secured. This finding has led to the accomplishment of the present invention.

### DISCLOSURE OF THE INVENTION

The present invention relates to a method of using an antacid for preventing sucralfate from adsorbing or trapping other drugs. The invention also relates to a pharmaceutical preparation comprising sucralfate, another drug and an antacid in such a way that the another drug is prevented from being adsorbed on or trapped by sucralfate.

### BRIEF DESCRIPTION OF DRAWINGS

Fig. 1 shows release curves for famotidine from the capsules prepared in Comparative Example 1 and Reference Example 1 in 900-mL of the Japanese Pharmacopoeia (JP) first solution.
Fig. 2 shows plasma level curves for famotidine in the capsules of Comparative Example 1 and Reference Example 1 which were each administered to 10 beagles.
Fig. 3 shows release curves for famotidine in 500-mL of the JP first solution containing the capsules prepared in Examples 1 and 2, Comparative Example 2 and Reference Example 1.
Fig. 4 shows plasma level curves for famotidine in the capsules of Examples 1 and 2 and Reference Example 1 which were each administered to 10 beagles.
Fig. 5 shows release curves for famotidine from the capsules prepared in Examples 1 and 3 and Comparative Example 2 in 500-mL of the JP first solution.
Fig. 6 shows release curves for cimetidine from the capsules prepared in Example 4 and Comparative Example 3 in 500-mL of the JP first solution.
Fig. 7 shows a release curve for famotidine from the tablet prepared in Example 5 in a 200-mL of the JP first solution.
Fig. 8 shows plasma level curves for famotidine in the tablets of Example 6 and Reference Example 2 which were each administered to 9 beagles.

### MODES FOR CARRYING OUT THE INVENTION

The antacid to be used in the invention method of use may be used along with a pharmaceutical preparation containing sucralfate and another drug; alternatively, it may be first used along with either one of sucralfate and another drug and then mixed with either the other of sucralfate and another drug. In the pharmaceutical preparation of the invention, the antacid is preferably located at the same site as sucralfate but it need not be located at the same site as the another drug and may be located at a different site insofar as they are within the same pharmaceutical preparation. For example, sucralfate may be mixed with another drug and the antacid to form a pharmaceutical preparation or it may be first mixed with the antacid to form a pharmaceutical preparation, to which a pharmaceutical preparation of another drug is added to make a single pharmaceutical preparation. Alternatively, a pharmaceutical preparation may be formed from two separate layers, one consisting of a mixture of sucralfate and the antacid and the other containing another drug.

The expression "adsorption or trapping of another drug" as used in the present invention refers to such circumstances that due to various causes such as the adsorption of the drug on sucralfate in the digestive tract, its trapping in a gel or suppressed absorption due, for example, to the formation of a complex, drug interactions occur as exemplified by inhibition of or delay in the intended adsorption of the another drug, as well as the decrease in its efficacy.

According to the invention, the adsorption of another drug on sucralfate in the digestive tract can be prevented without containing said drug and sucralfate in separate layers. In addition, the incorporated antacid delays the gelation of sucralfate in the digestive tract, thereby preventing the drug from being trapped within the sucralfate gel. As a further advantage, the formation of a complex of the drug and sucralfate due to their interaction can be prevented or delayed. Because of these advantageous effects, the intended absorption of the another drug to be used along with sucralfate can be prevented from being inhibited or delayed and, what is more, the occurrence of drug interactions such as the decrease in its efficacy can be prevented.

The another drug to be used in the invention is not limited to any particular types in such aspects as structure and efficacy and may be exemplified by the following which can be used either individually or in combination: antiepileptics such as phenytoin; antipyretics and analgesics such as ketoprofen, ibuprofen, naproxen and indomethacin; psychopharmacologic agents such as chlorpromazine hydrochloride and sulpiride; antispasmodic drugs such as methylbenactyzium bromide and amitriptylin hydrochloride; cardiotonics such as digoxin; antidysrhythmic drugs such as procainamide hydrochloride, indenolol hydrochloride and qunididine sulfate; bronchodilators such as theophylline; H₂-receptor antagonists such as cimetidine, ranitidine, famotidine, nizatidine and loxatidine; anticoagulants such as warfarin potassium; antidiabetic drugs such as chlorpropamide; and antimicrobials such as aluminoparaaminosalicylic acid calcium salt, ciprofloxan hydrochloride, norfloxacin and ketoconazole. Particularly suitable for use are those drugs which, when administered simultaneously with sucralfate, will be adsorbed or trapped by sucralfate to experience reduced absorption.

Among the examples of the another drug to be used in the invention, H₂-receptor antagonists such as cimetidine, ranitidine, famotidine, nizatidine and loxatidine are drugs that may be used with particular advantage in the invention and, in particular, famotidine may be used in a further preferred embodiment.

The antacid to be used in the invention is desirably an inorganic antacid. Suitable examples include synthetic hydrotalcite, magnesium metasilicate aluminate, magnesium silicate aluminate, dry aluminum hydroxide gel, magnesium oxide, magnesium carbonate, precipitated calcium carbonate, sodium hydrogencarbonate and magnesium oxide. Amino acid antacids such as aminoacetic acid that are not inorganic antacids are not suitable for use in the present invention.

The amount of the antacid to be used in the invention ranges from 1:20 to 2:1 in terms of weight ratio to sucralfate. More preferably, it ranges from 1:10 to 1:1. If the amount of the antacid is less than the stated ranges, its effectiveness against a sucralfate gel may potentially fail to develop.

The amount of another drug to be incorporated along with sucralfate in the invention is not limited to any particular value but the another drug, if it is incorporated in a smaller amount than sucralfate, will benefit from the advantage of the invention in a particularly preferred way. If the another drug is incorporated in a larger amount than sucralfate, the relative intensity of suppression of its absorption by sucralfate is inherently small and would be substantially free of any adverse effect. Under the circumstances, the another drug is effectively incorporated in an amount not exceeding about one fifth of the weight of the sucralfate to be incorporated simultaneously.

The sucralfate to be used in the invention may be commercially available as under the trade name of "Ulcerlmin" (registered trademark of Chugai Pharmaceutical Co., Ltd.) or it may be obtained by synthesis procedures. If sucralfate is to be synthesized, it can be produced by known processes such as those described in Japanese Patent Publication Nos. 11673/1969, 16037/1969 and 76927/1993 and International Publication W09204030. If desired, the yet to be dried particles that are obtained from various synthesis procedures or sucralfate prepared by drying them may also be used.

The pharmaceutical preparation of the invention may be used in various dosage forms including capsules, granules, fine granules, powdered drugs, powders, pills, chewables, troches, suppositories, solutions, suspensions, emulsions, lotions, ointments, cataplasms, elixirs and so forth; dosage forms of any other structures will do insofar as the present invention can be implemented. There are also no limitations on pharmaceutical formulation techniques and commonly employed methods will do as exemplified by high shear granulation, fluidized bed granulation, freeze drying and so forth; if desired, dosage forms of interest may be subjected to film coating. Additives that can be used in the pharmaceutical preparation are not limited, either and those which are employed in the making of ordinary medical drugs will do as exemplified by vehicles such as crystalline cellulose and lactose, disintegrators such as corn starch and croscarmellose, binders such as hydroxypropyl cellulose and lubricants such as magnesium stearate. Other auxiliary agents that can be added include dispersing agents, fillers, excipients, solvents, emulsifiers, antiseptics, scents, moistening agents, flavoring agents, dyes, buffers and so forth.

On the pages that follow, reference examples and working examples are provided to further illustrate the present invention but are in no way to be taken as limiting.

### Comparative Example 1

### Capsule:

| | |
|---|---|
| Famotidine | 10 parts |
| Sucralfate | 1000 parts |

The indicated amounts of ingredients were weighed and mixed well; thereafter, gelatin capsules of size No. 000 were filled with the mixture one capsule for a single does of 1010 mg.

### Reference Example 1

### Capsule:

| | |
|---|---|
| Famotidine | 10 parts |
| Lactose | 800 parts |

The indicated amounts of ingredients were weighed and mixed well; thereafter, gelatin capsules of size No. 000 were filled with the mixture, 810 mg per capsule. In addition, gelatin capsules of size No. 00 were filled with the mixture, 405 mg per capsule.

### Test 1

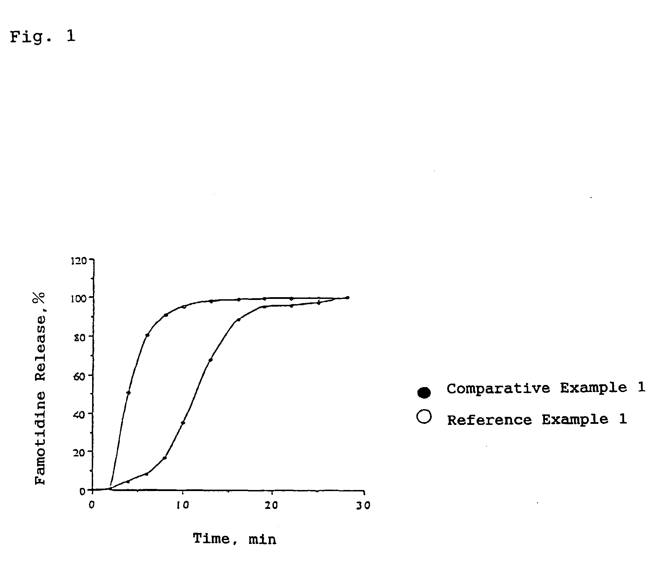

One each of the capsules of size No. 000 prepared in Comparative Example 1 and Reference Example 1 was tested for dissolution by the paddle method described in the JP using 900 mL of the JP first fluid. The results are shown in Fig. 1.

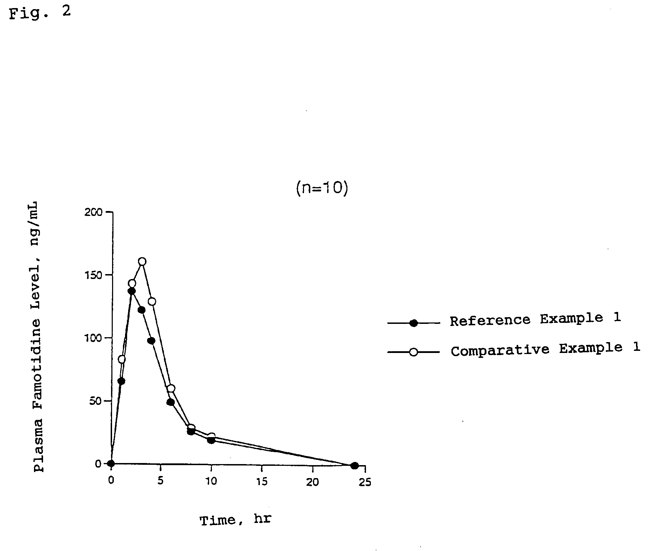

One each of the capsules of size No. 000 prepared in Comparative Example 1 and Reference Example 1 was administered with 50 mL of water by the crossover method to 10 beagles (male weighing about 14 kg on average) that had been fasted overnight; 10-mL blood samples were taken at time intervals and the plasma famotidine level was measured by HPLC to construct blood level curves. The results are shown in Fig. 2.

The results of the dissolution test shown in Fig. 1 demonstrate that the capsule of Comparative Example 1 having famotidine incorporated along with sucralfate experienced delayed release of famotidine in the first fluid.

Further, from the results of the absorption test on the beagles that are shown in Fig. 2, it was seen that the capsule of Comparative Example 1 having famotidine incorporated along with sucralfate experienced less famotidine absorption than the capsule of Reference Example 1, demonstrating a significant difference in AUC at a 5% risk.

### Example 1

### Capsule:

| | |
|---|---|
| Famotidine | 10 parts |
| Sucralfate | 750 parts |
| Synthetic hydrotalcite | 250 parts |

The indicated amounts of ingredients were weighed and mixed well; thereafter, gelatin capsules of size No. 00 were filled with the mixture 505 mg per capsule.

### Example 2

### Capsule:

| | |
|---|---|
| Famotidine | 10 parts |
| Sucralfate | 750 parts |
| Synthetic hydrotalcite | 150 parts |
| Sodium hydrogencarbonate | 200 parts |

The indicated amounts of ingredients were weighed and mixed well; thereafter, gelatin capsules of size No. 00 were filled with the mixture, 555 mg per capsule.

### Comparative Example 2

### Capsule:

| | |
|---|---|
| Famotidine | 10 parts |
| Sucralfate | 750 parts |

The indicated amounts of ingredients were weighed and mixed well; thereafter, gelatin capsules of size No. 00 were filled with the mixture, 380 mg per capsule.

### Test 2

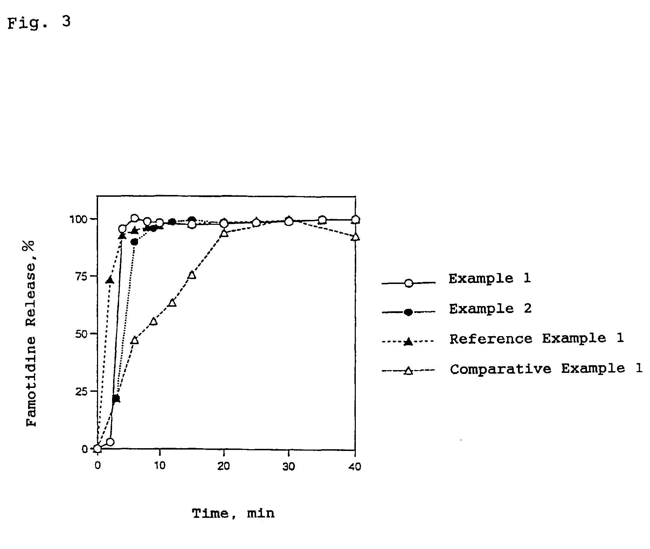

Two each of the capsules of size No. 00 prepared in Examples 1 and 2, Comparative Example 2 and Reference Example 1 were tested for dissolution by the paddle method described in the JP using 500 mL of the JP first fluid. The results are shown in Fig. 3.

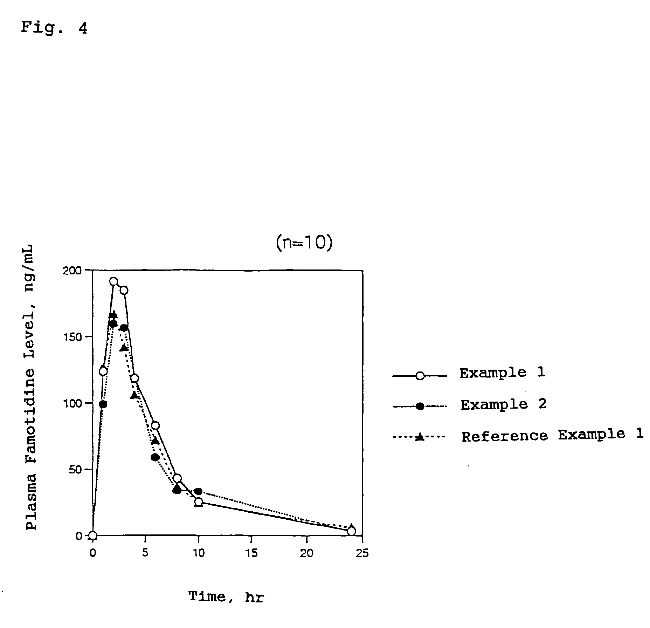

Two each of the capsules of size No. 00 prepared in Examples 1 and 2 and Reference Example 1 were administered with 50 mL of water by the Latin square method to 10 beagles (male weighing about 10 kg on average) that had been fasted overnight; 5-mL blood samples were taken at time intervals and the plasma famotidine level was measured by HPLC to construct blood level curves. The results are shown in Fig. 4.

The results of the dissolution test shown in Fig. 3 demonstrate that the capsules of Comparative Example 2 having famotidine incorporated along with sucralfate experienced delayed release of famotidine whereas the capsules of Examples 1 and 2 which also contained an antacid exhibited as fast famotidine release in the first fluid as the capsules of Reference Example 1.

From the results of the absorption test on the beagles that are shown in Fig. 4, it can be seen that the variance analysis of AUC among Example 1, 2 and Refernce Example 1 showed no significant differences at a 60% power, indicating comparable levels of famotidine absorption; thus, the incorporation of the antacid was effective in avoiding the suppressed absorption of famotidine. The two tests described above support the assumption that the evaluation of the difference in absorbability can be predictably evaluated by conducting a dissolution test in the first fluid and, therefore, in some of the tests to be described below, evaluation was performed by a dissolution test.

### Example 3

### Capsule:

| | |
|---|---|
| Famotidine | 10 parts |
| Sucralfate | 750 parts |
| Synthetic hydrotalcite | 75 parts |

The indicated amounts of ingredients were weighed and mixed well; thereafter, gelatin capsules of size No. 00 were filled with the mixture 417.5 mg per capsule.

### Test 3

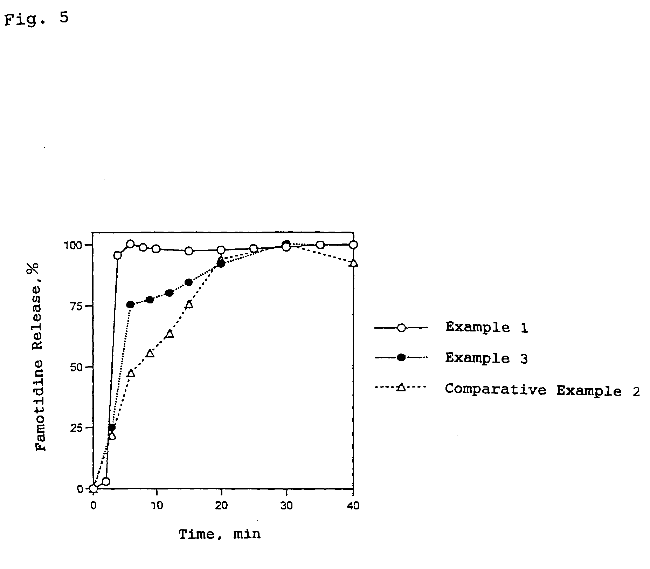

Two each of the capsules of size No. 00 prepared in Example 3 were tested for dissolution by the paddle method described in the JP using 500 mL of the JP first fluid. The results are shown in Fig. 5.

From the results of the dissolution test shown in Fig. 5, it can be seen that although the capsules of Example 3 incorporating the antacid in an amount one tenth the weight of sucralfate were somewhat delayed in the release of famotidine, the dissolution in the in first fluid was improved compared to the capsules of Comparative Example 2.

### Example 4

### Capsule:

| | |
|---|---|
| Cimetidine | 100 parts |
| Sucralfate | 1000 parts |
| Sodium hydrogencarbonate | 100 parts |

The indicated amounts of ingredients were weighed and mixed well; thereafter, gelatin capsules of size No. 00 were filled with the mixture, 600 mg per capsule.

### Comparative Example 3

### Capsule:

| | |
|---|---|
| Cimetidine | 100 parts |
| Sucralfate | 1000 parts |

The indicated amounts of ingredients were weighed and mixed well; thereafter, gelatin capsules of size No. 00 were filled with the mixture, 550 mg per capsule.

### Test 4

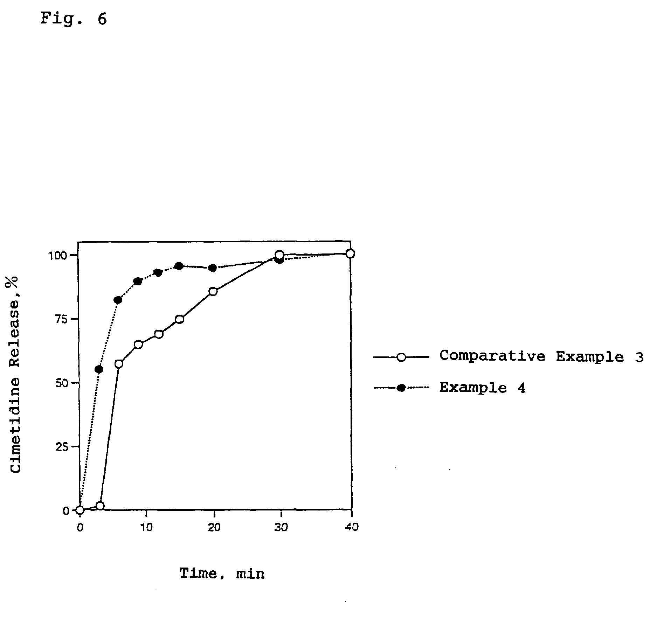

Two each of the capsules of size No. 00 prepared in Example 4 and Comparative Example 3 were tested for dissolution by the paddle method described in the JP using 500 mL of the JP first fluid. The results are shown in Fig. 6.

From the results of the dissolution test shown in Fig. 6, it can be seen that the capsules of Example 4 incorporating the antacid allowed cimetidine to be released faster in the first fluid than the comparative capsules incorporating no antacid.

### Example 5

### Tablet:

| | |
|---|---|
| Famotidine | 100 parts |
| Sucralfate | 1000 parts |
| Magnesium metasilicate aluminate | 250 parts |
| Crystalline cellulose (Avicel PH101) | 234 parts |
| Carboxymethyl starch sodium | 75 parts |
| Hydrogenated oil | 34 parts |
| Magnesium stearate | 2 parts |
| Hydrous amorphous silicon oxide | 5 parts |

The indicated amounts of ingredients were weighed and mixed well; thereafter, the mixture was compressed into flat-face tablets of 9.5mm^{⌀} (each weighing 425 mg) at a total pressure of 1 t; the tablets had a hardness of 23 kg.

### Test 5

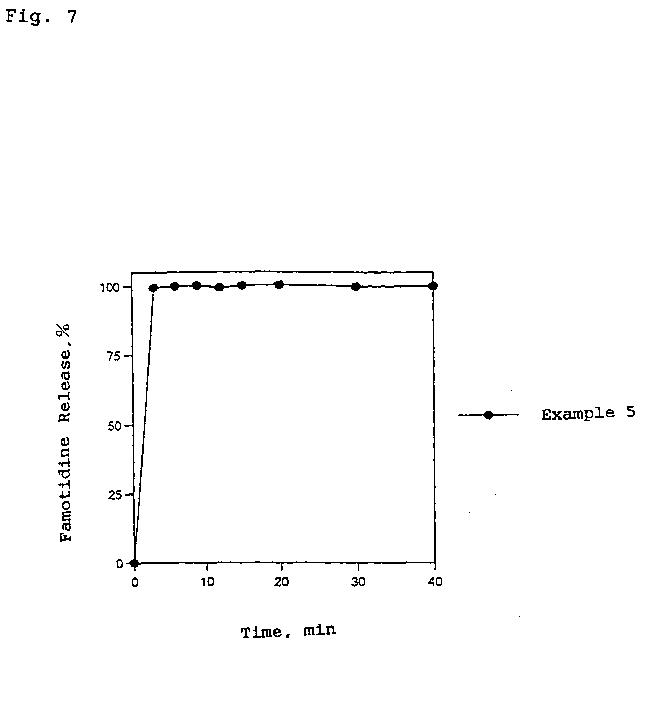

One of the tablets of Example 5 was put into 200 mL of the JP first fluid in a beaker at 37°C under agitatation, the supernatant was sampled at time intervals and the filtered sample was measured for absorbance with a spectro-photometer. The result is shown in Fig. 7.

From the result of the dissolution test shown in Fig. 7, it can be seen that famotidine was also released from the tablet at fast rate in the first fluid.

### Example 6

### Tablet:

| | |
|---|---|
| Famotidine | 10 parts |
| Sucralfate | 750 parts |
| Synthetic hydrotalcite | 250 parts |
| Crystalline cellulose (Aveicel PH101) | 86 parts |
| Corn starch | 24 parts |
| Hydroxypropyl cellulose | 174 parts |
| Magnesium stearate | 6 parts |

The indicated amounts of ingredients were weighed and mixed well; the mixture was compressed into flat-face tablets of 12 mm^{⌀} (each weighing 650 mg) at a total pressure of 2 t; the tablets had a disintegration time of 1.9 min in water.

### Reference Example 2

### Tablet:

| | |
|---|---|
| Famotidine | 10 parts |
| Mannitol | 1000 parts |
| Crystalline cellulose (Avicel PH101) | 86 parts |
| Corn starch | 24 parts |
| Hydroxypropyl cellulose | 174 parts |
| Magnesium stearate | 6 parts |

The indicated amounts of ingredients were weighed and mixed well; the mixture was compressed into flat-face tablets of 12 mm^{⌀} (each weighing 650 mg) at a total pressure of 3 t; the tablets had a disintegration time of 1.6 min in water.

### Test 6

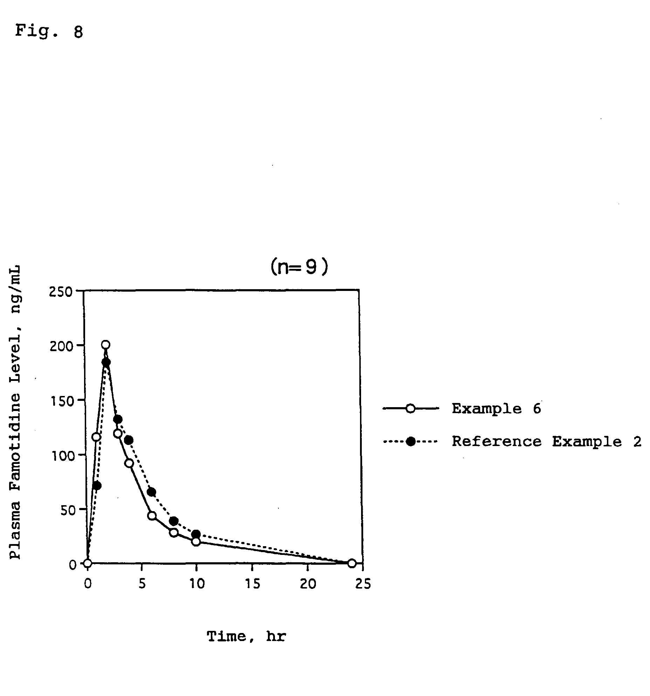

Two each of the tablets of Example 6 and Reference Example 2 were administered with 50 mL of water by the crossover method to 9 beagles (male weighing 12 kg on average) that had been fasted overnight; 10-mL blood samples were taken at time intervals and the plasma famotidine level was measured by HPLC to construct blood level curves. The results are shown in Fig. 8.

From the results in Fig. 8, it can be seen that the tablets of Example 6 incorporating not only sucralfate but also the antacid had no significant differences in Cmax and AUC from the tablets of Reference Example 2 which were fast-release famotidine preparations; hence, the absorption of famotidine was comparable between the two kinds of tablet.

## Claims

1. A method of using an antacid for preventing sucralfate from adsorption or trapping of another drug.

2. The method according to claim 1, wherein the antacid is used in an amount ranging from 1:20 to 2:1 in weight ratio to sucralfate.

3. The method according to claim 2, wherein the antacid is used in an amount ranging from 1:10 to 1:1 in weight ratio to sucralfate.

4. The method according to any one of claims 1 - 3, wherein the antacid is an inorganic antacid.

5. The method according to any one of claims 1 - 4, wherein the antacid is at least one inorganic antacid selected from the group consisting of synthetic hydrotalcite, magnesium metasilicate aluminate, magnesium silicate aluminate, dry aluminum hydroxide gel, magnesium oxide, magnesium carbonate, precipitated calcium carbonate, sodium hydrogencarbonate and magnesium oxide.

6. The method according to any one of claims 1 - 5, wherein the another drug is a drug that is adsorbed on or trapped by the incorporated sucralfate to experience reduced absorption.

7. The method according to any one of claims 1 - 6, wherein the another drug is an antiepileptic phenytoin, an antipyretic and analgesic ketoprofen, ibuprofen, naproxen or indomethacin, a psychopharmacologic agent chlorpromazine hydrochloride or sulpiride, an anti-spasmodic drug methylbenactyzium bromide or amitriptylin hydrochloride, a cardiotonic digoxin, an antidysrhythmic drug procainamide hydrochloride, indenolol hydrochloride or qunidine sulfate, a bronchodilator theophylline, a H₂-receptor antagonist cimetidine, ranitidine, famotidine, nizatidine or loxatidine, an anticoagulant warfarin potassium, an antidiabetic drug chlorpropamide, an antimicrobial aluminoparaaminosalicylic acid calcium salt, ciprofloxan hydrochloride, norfloxacin or ketoconazole, or any one of the combinations thereof.

8. The method according to claim 7, wherein the another drug is a H₂-receptor antagonist.

9. The method according to claim 7 or 8, wherein the another drug is a H₂-receptor antagonist selected from among cimetidine, ranitidine, famotidine, nizatidine and loxatidine acetate.

10. The method according to claim 9, wherein the another drug is famotidine.

11. A pharmaceutical preparation comprising sucralfate, another drug and an antacid, the another drug being prevented from being adsorbed on or trapped by sucralfate.

12. The pharmaceutical preparation comprising sucralfate, another drug and an antacid is adapted to have a capability of preventing the another drug from being adsorbed on or trapped by sucralfate.

13. The pharmaceutical preparation according to claim 11 or 12, wherein the antacid and sucralfate are incorporated in a weight ratio ranging from 1:20 to 2:1.

14. The pharmaceutical preparation according to claim 13, wherein the antacid and sucralfate are incorporated in a weight ratio ranging from 1:10 to 1:1.

15. The pharmaceutical preparation according to any one of claims 11 - 14, wherein the antacid is an inorganic antacid.

16. The pharmaceutical preparation according to any one of claims 11 - 15, wherein the antacid is at least one inorganic antacid selected from the group consisting of synthetic hydrotalcite, magnesium metasilicate aluminate, magnesium silicate aluminate, dry aluminum hydroxide gel, magnesium oxide, magnesium carbonate, precipitated calcium carbonate, sodium hydrogencarbonate and magnesium oxide.

17. The pharmaceutical preparation according to any one of claims 11 - 16, wherein the another drug is a drug that is adsorbed on or trapped by the incorporated sucralfate to experience reduced absorption.

18. The pharmaceutical preparation according to any one of claims 11 - 17, wherein the another drug is an antiepileptic phenytoin, an antipyretic and analgesic ketoprofen, ibuprofen, naproxen or indomethacin, a psychopharmacologic agent chlorpromazine hydrochloride or sulpiride, an antispsmodic drug methybenactyzium bromide or amitroptylin hydrochloride, a cardiotonic digoxin, an antidysrhythmic drug procainamide hydrochloride, indenolol hydrochloride or qunidine sulfate, a bronchodilator theophylline, a H₂-receptor antagonist cimetidine, ranitidine, famotidine, nizatidine or loxatidine, an anticoagulant warfarin potassium, an antidiabetic drug chlorpropamide, an antimicrobial aluminoparaaminosalicylic acid calcium salt, ciprofloxan hydrochloride, norfloxacin or ketoconazole, or any one of the combinations thereof.

19. The pharmaceutical preparation according to claim 18, wherein the another drug is a H₂-receptor antagonist.

20. The pharmaceutical preparation according to claim 18 or 19 wherein the another drug is a H₂-receptor antagonist selected from among cimetidine, ranitizine, famotidine, nizatidine and loxatidine acetate.

21. The pharmaceutical preparation according to claim 20, wherein the another drug is famotidine.
